# EUROPEAN PATENT SPECIFICATION

(11) **EP 0 857 791 B1**
(45) Date of publication and mention of the grant of the patent: **31.03.2004**
(21) Application number: 97122253.4
(22) Date of filing: 17.12.1997
(51) Int. Cl.: C12Q 1/68

(54) **Method of analysis using signal amplification**
Analyseverfahren basierend auf Signalverstärkung
Méthode d'analyse utilisant l'amplification des signaux

(30) Priority: 21.12.1996 EP 96120730
(43) Date of publication of application: 12.08.1998
(73) Proprietor: PNA Diagnostics A/S, 2600 Glostrup (DK)
(72) Inventor: Orum, Henrik, Dr., 3500 Vorlose (DK); Koch, Troels, Dr., 2300 Kobenhaven S. (DK); Borre, Martin, Dr., 2640 Hedehusene (DK); Hansen, Henrik Frydenlund, 2660 Rodovre (DK)
(74) Representative: Christiansen, Ejvind

(56) References cited:
- WO-A-92/11390
- WO-A-95/01370
- WO-A-95/14706
- WO-A-95/20320
- IRO T ET AL: "SEQUENCE-SPECIFIC DNA PURIFICATION BY TRIPLEX AFFINITY CAPTURE" PROCEEDINGS OF THE NATIONAL ACADEMY OF SCIENCES OF USA, vol. 89, 1 January 1992, pages 495-498, XP002000128

## Description

### Field of the invention

The present invention relates to chimeric nucleic acids compounds that have the capacity of forming triplex structures, to the production and purification of such chimeric compounds, and to their use in signal amplification techniques.

### Background of the invention

Detection and quantification of nucleic acid molecules constitutes a fundamental trait in several diagnostic techniques. However, the amount of accessible nucleic acid target molecules in a specific sample is in general low, and direct hybridization with a polynucleotide probe therefore in many cases is insufficient for detection. In the case of an infectious organism, the classical solution has been enrichment by culturing, but this is time-consuming and therefore unsuitable for rapid diagnosis. A way to circumvent these problems is to amplify the target molecule. This can be accomplished in one of several ways, for example polymerase chain reaction (PCR, as it is described in US-A-4,683,195 or EP-A-0 630 971), ligase chain reaction (LCR, EP-A-0 320 308) or NASBA (EP-A-329 822). However, all of these techniques utilize enzymes for amplification of target nucleic acid molecules and, consequently, they suffer from the drawback that compounds in the sample specimen may inhibit these enzymes. Therefore, current protocols based on these techniques often include laborious sample preparation steps such as, for example purification and concentration of DNA. Furthermore, when the target sequence is amplified the risk of contamination becomes significantly increased. Several methods have been described to overcome contamination problems, including 1) digestion with nucleases that specifically degrades nucleic acid molecules that have been produced during previous analysis (EP-A-0 401 037); and 2) special laboratory practice that minimises carry over between new samples and previously analysed material. However, methods for avoiding contamination and inhibition are in general costly and cumbersome.

Another solution for signal amplification would be to employ a method in which the number of signal-producing compounds is increased. Such methods have been described by for example EP-A-0 317 077, WO 95/01365 and WO 95/08000. These techniques utilize multivalent intermediate probes containing a large amount of identical nucleotide sequences bound covalently to each other and each intermediate probe being capable of hybridizing with up to 50 signal producing secondary probes simultaneously. However, these techniques utilize large molecules as intermediate probes which results in less favorable kinetics.

Thus, it would be highly desirable to have a method for signal amplification which is rapid, simple, sensitive, based on stable compounds and on non-enzymatical reactions.

From WO 92/11390 A1, triple helix structures are known. Such triple helix structures are formed by binding of a so-called "triplex probe" to a duplex, i.e. the triple helix structure are formed by attachment to or by strand displacement of a duplex. The present invention differs in that multiple probe molecules are used containing a segment designed for binding to the molecule to be determined, and a part designed to minimise binding by base pairing to the same probe molecule, whereby triple helix structures are formed.

### Summary of the invention

From PNAS USA Vol. 89, 495-498 (1992), triple helix structures are known. Such triple helix structures are formed by intermolecular triplex formation between a double-stranded sequence and a probe. The present invention differs in that multiple probe molecules are used comprising a segment designed for binding to the molecule to be determined, and a part designed to minimise binding by base pairing to the same probe molecule, whereby triple helix structures are formed.

Subject of the invention is therefore a method of determining a molecule A containing a sequence SA of nucleobases, said molecule being capable of forming a triple helix structure through said sequence SA comprising providing a reaction mixture containing multiple probe molecules B containing a first segment B1 capable of participating in a triple helix structure with A through sequence SA and a segment B2 that allows for direct or indirect detection, incubating said reaction mixture at conditions allowing the formation of triple helices and determining the formation of triple helices using said segment B2.

A further subject of the invention is a molecule especially advantageous for this method.

A further object of the invention is a method for the determination of an analyte using the above mentioned method for determining a molecule A.

A further object of the invention is the use of multiple triple helix structures (triplex structures) for the determination of an analyte.

### Brief description of the drawings

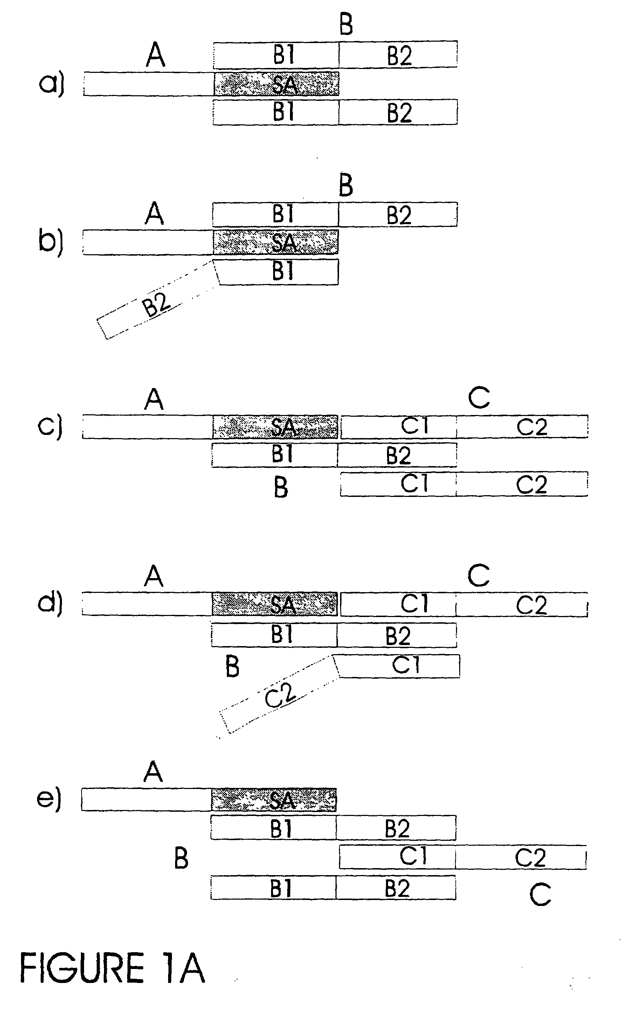

Figure 1A is a schematic illustration of structures formed from molecules A and probes B.

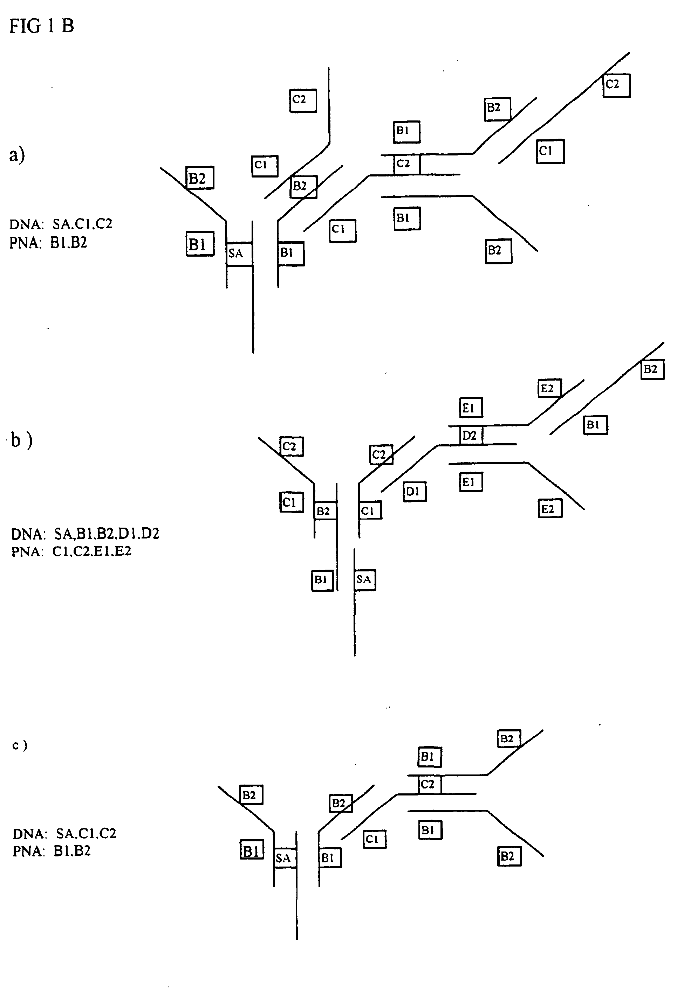

Figure 1B is a schematic illustration of structures showing how the structures of Figure 1A can be extended further.
Figure 2 is a schematic illustration of a DNA and a DNA molecule
In Figure 3 several ways to link probe segments are given. In the case of the segments comprising DNA and PNA formation of an intramolecular duplex is favored in case A and B. This means that cases C and D are preferred arrangements for forming triple helix structures. Figure 3 contains the nomenclature used throughout the specification for the arrangement and orientation of strands.

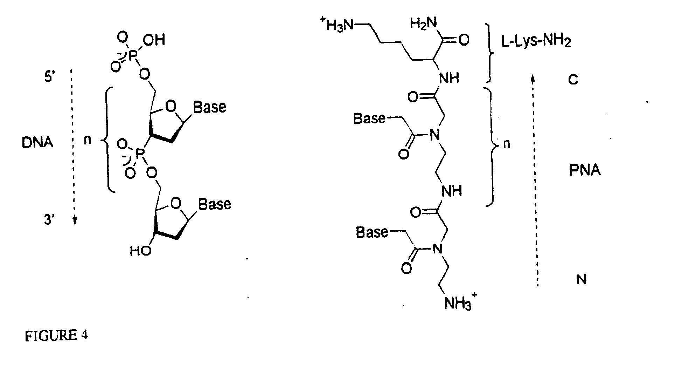

Figure 4 shows schematically the structure of an exemplified PNA while bound to DNA in the preferred antiparallel mode. It further shows that nucleic acid analogues can be modified by the attachment of groups improving the solubility of the probe, in case of PNA the addition of a lysine residue.
Figure 5 gives a schematic picture of a construct formed in one of the exemplified methods according to the invention, wherein a target nucleic acid is analyzed using a primary probe, multiple probes composed of each a DNA and a PNA segment and secondary probes containing a reporter group, and having a blocked end that disables the probe in participating in further assembly. It shows that using the invention it is possible to obtain a multiplicity of labeling sites and therefore achieve signal amplification.
In Figure 6A a schematic picture is shown for an exemplified embodiment of the invention using a first probe B containing two segments B1 and B2, and a second probe C containing two segments C1 and C2. The segments B1 and C1 consist of PNA and the segments B2 and C2 consists of DNA.
Figure 6B shows the result of an assembly performed as in 6A but in the presence of four probes, termed B, C , D and E containing two segments each. Both segments of probes C and E consist of DNA, and both segments of probes B and D consist of PNA.
Figure 7 shows a sequence and binding mode of one molecule of probe C and two molecules of probe D, wherein the second segment of probe D can bind the first segment of probe C.
Figure 8 shows schematically a probe molecule containing two PNA segments, the amino ends being bound together covalently by a linker moiety L.
Figure 9 shows the mode in which two PNA molecules bind to one strand of DNA.
Figure 10 shows an assembly containing only one molecule B. In this case, B 1 is PNA and B2 is nucleic acid.

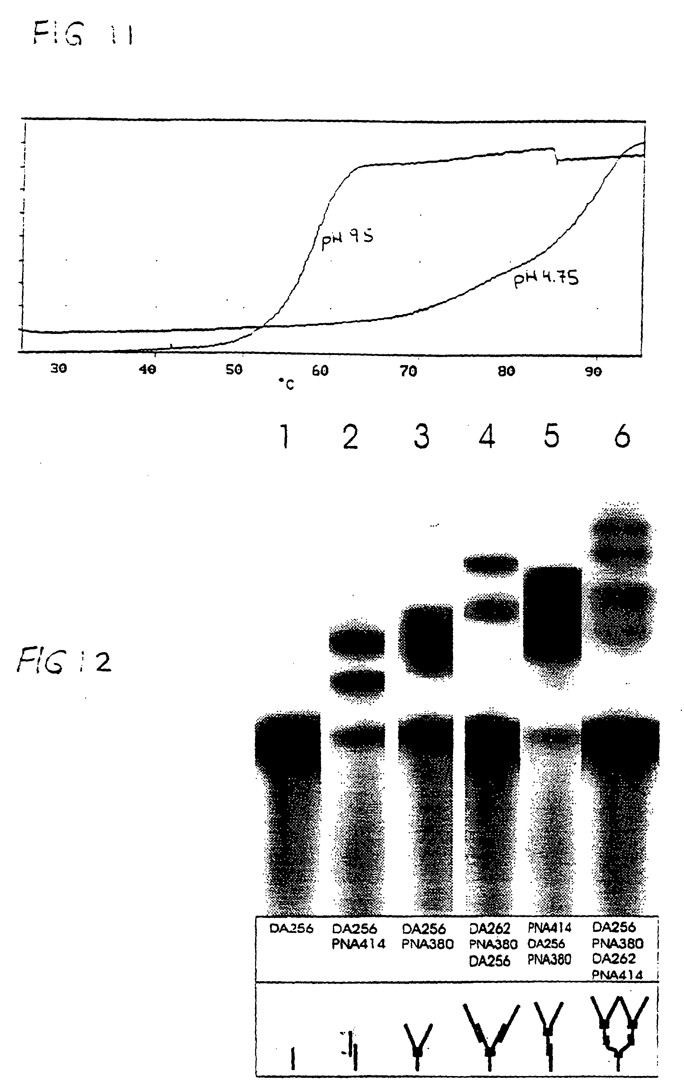

Figure 11 shows the melting curves of complexes formed between the oligos of example 1 (denoted B1 and C2), measured at pH 4.75 and pH 9.5.
Figure 12 shows an autoradiogram showing the different starting compounds and reaction products for the formation of duplexes and triplexes.
Figure 13 shows an autoradiogram for the formation of a complex involving duplexes and a triplex, wherein the amount of the only duplex forming oligonucleotide is increased from lane 1 to lane 7 (0; 1; 5; 10; 20; 40; 100 pmol DA262). Lane 8 contains only the triplex forming component DA256.

### Detailed description of the invention

A molecule A can be any moiety containing a sequence SA of nucleobases which sequence is preferably a least 9 bases long, most preferably is between 10 and 30 bases long. The nucleobases are preferably covalently linked in a linear manner to a backbone, especially in a way such that the molecule can bind by base pairing to a nucleic acid containing a sequence of bases complementary to the sequence of bases contained in molecule A. Examples of molecules A are nucleic acids, like RNA, DNA or derivatives thereof and nucleic acid analogues. In very preferred manner the nucleic acid is DNA. Molecule A can also be a molecular moiety, for example a protein or hapten to which a nucleic acid or nucleic acid analogue sequence is attached.

A can be a molecule originating from a sample wherein the presence, absence or amount of this molecule is intended to be determined and analyzed in presence/absence or/and amount, but molecule A can also be a probe or probe unit that is itself intended to be used for the determination of an analyte. Generally the intention behind the occurrence or use of the sequence SA in molecule A is to increase the number of binding sites for labels or the number of labels attached or attachable to molecule A. Therefore the origin and function of the part of molecule A not participating in amplifying a signal can be chosen according to the specific task to be solved. Further the amount of molecule A can be unknown, for example in methods for determining molecule A as an analyte in a sample, or it can be known, for example if a defined amount of molecules A is added to a sample, especially for determining an analyte (for example antigens, antibodies, haptens etc.) different from molecule A.

Sequence SA can contain natural nucleobases, like A, C, G, T and U, or non-natural bases, like 7-deaza-G and mixtures. The important feature of sequence SA is that it has the ability to bind a probe molecule B by base pairing.

A triple helix structure or triplex structure is composed of three strands of molecules, each containing a nucleobase sequence capable of base pairing. Preferably the mode of binding between the three strands involves both Watson/Crick and Hoogsteen base pairing. The formation of triple helix structures requires a high degree of complementarity of the sequence of two of the strands involved to the third strand. In preferred triple helix structures two of the strands are composed of pyrimidine nucleobases of any sequence while the third strand is composed of the complementary purine nucleobase sequence. The triple helical structure therefore preferably has a length of at least six purine or six pyrimidine nucleobases respectively.

It has been shown in WO 95/01370 that peptide nucleic acids (PNAs) have the ability to form triplex structures with nucleic acids. Criteria for forming triple helices can therefore be taken from this patent application. PNAs and their synthesis are disclosed in WO 92/20702 and WO 94/25477. In one embodiment the backbone of PNAs is composed of repeating units of ethylaminoglycine moieties, wherein the nucleobases are bound to the glycine amino group. PNAs in this definition therefore contain an amino terminus (-NH2) and a carboxylic acid terminus (-COOH). These termini can be modified by the attachment of other moieties or the omission of groups (for example the linkage to groups increasing solubility, like lysine).

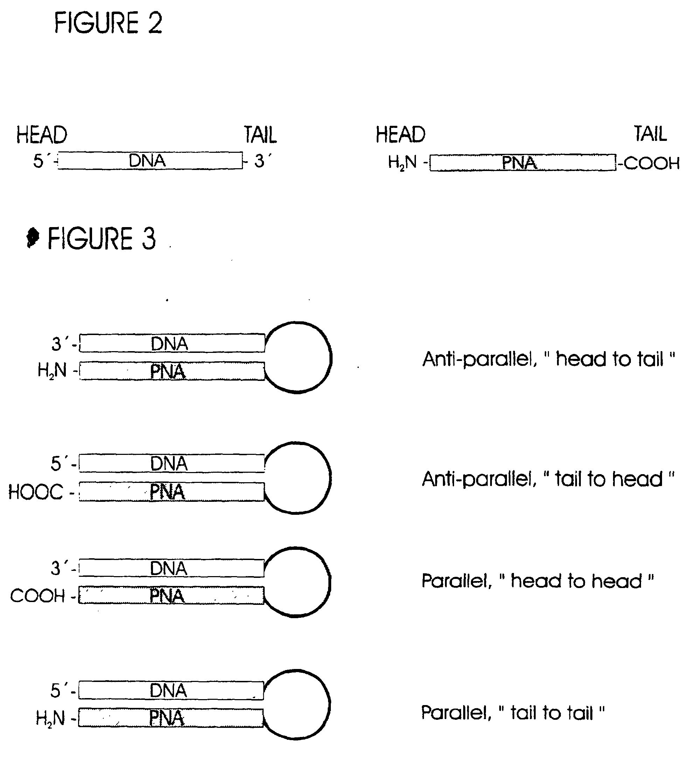

In Figure 3 there are shown four possible cases of intramolecular base pairing between a DNA and PNA part of one probe molecule. Prerequisite to Figure 3 is that the PNA and the DNA part are able to hybridize by base pairing. It becomes clear that the way of linking the PNA sequence to a DNA sequence as shown in Figures 3C and 3D is favored over the embodiments shown in Figures 3A and 3B. Case 3C and 3D are referred to as parallel binding. In case A the 5' terminus of the DNA (head) is bound to the carboxyl end of the PNA (tail). For forming triple helix structures, the antiparallel arrangement of the sequences should therefore be avoided, the parallel arrangement should be preferred.

A probe according to the invention is an entity used for binding a label of any kind to a molecule to be determined. This binding may be direct or indirect.

An assembly of the present invention is defined to contain at least one, preferably just one, molecule A and a multiplicity of different identical probe molecules, preferably more than 2 and most preferably between 10³ and 10⁷ probe molecules.

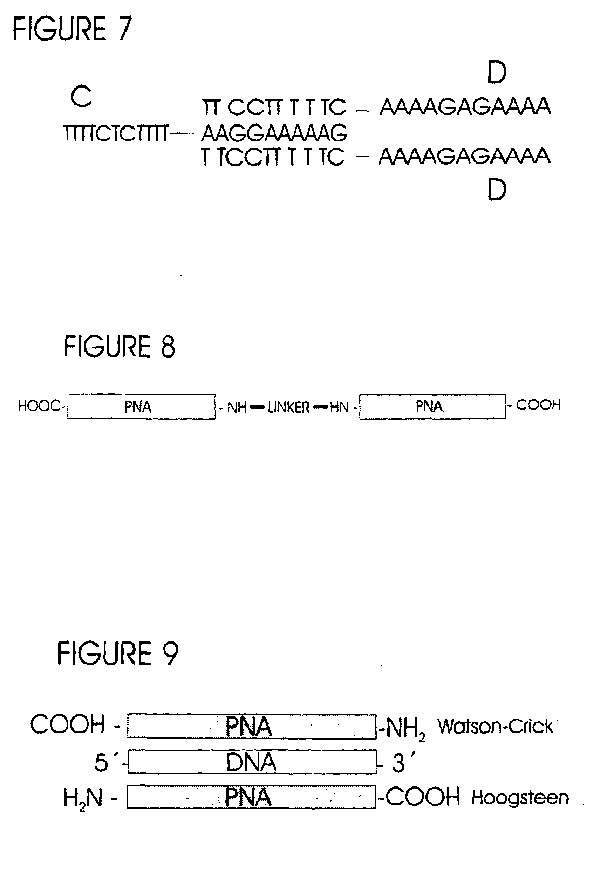

Each probe molecule B is defined to contain at least two segments. They are named in the following as B1 and B2. These segments are preferably and most practically linked together covalently, either directly or indirectly via a linker moiety. It will become apparent from the detailed description of the invention, that the linkage of the two segments must essentially fulfill the purpose to fix the arrangements/orientation of the segments within each probe molecule and to space the regions between B1 and B2. The linker moiety L therefore may have the function to provide a chemical entity for connecting the termini of the segments, but may further have the function to provide a defined distance between the segments to be linked. Linker moieties according to the invention are bivalent moieties of 1 to 100 atoms or more. Typical linker moieties are alkylene groups, dicarboxylic acid moieties, diamine moieties or amino acid moieties. Preferred distance is between 20 and 100 atoms, counted between the atoms following the first non-base atom of adjacent bases. One example of a probe molecule is shown in Figure 8. In this schematic presentation two PNA molecules, each having an amino and a carboxyl terminus are linked together by a linker moiety L, connecting two amino termini of the PNAs. Carboxylic acid termini of PNAs can for example be connected by the use of conventional condensing agents like diimides, for example 1-(3-dimethylaminopropyl)-3-ethyl carbodiimide (EDC). Rigid linkers are further advantageous to avoid intramolecular hybridization.

In case of a probe molecule containing a PNA and a DNA segment preferred linking moieties connect hydroxyl groups of the DNA segment with either of the amino or carboxyl end of the PNA segment (compare Figure 3). Molecules containing segments of nucleic acids and nucleic acid analogues are described in WO 95/08356 and WO 95/14706. These chimerical molecules can be used in the present invention as probe molecules. At least one of the segments of the probe molecule is capable of participating in a triple helix structure.

This segment is therefore preferably designed to consist of a sequence of nucleobases complementary to sequence SA of the molecule A.

By complementarity according to the invention there is meant more than 90% complementarity of a consecutive number of nucleobases of a length of at least nine bases. Complementarity is judged by hydrogen bonding between bases.

While segment B1 is designed for binding to molecule A, segment B2 is designed to minimize binding by base pairing to segment B1 of the same probe molecule (intramolecular binding). Therefore the arrangements of Figures 3C and 3D should be chosen. One further way to avoid intramolecular binding is by choosing a linker length, preferably small enough to sterically hinder such binding.

Conditions for formation of triple helical structures may vary dependent upon the kind of moieties making up sequence SA and segment B1. For PNAs and DNA the reaction conditions are described in WO 95/01370. These conditions can be applied for binding of molecule B to molecule A. Preferred these conditions include a pH less than 7, conferring protonation of cytosine molecules. For triple helical structures made up by other strands, a man skilled in the art can determine the optimal conditions for triplex formation by titration experiments or by comparing melting curves and association curves.

The triple helix structure formation reaction is allowed to proceed as long as required to form as much triple helices as intended which will generally be within 1 min. and 12 hours, preferably between 15 and 30 min. Generally, in a way to also estimate the amount of molecules A in the reaction mixture, for example in a quantitative assay, one would stop the reaction after a defined reaction time. Such stopping the reaction can be made by changing the pH such that no further triplex formation can occur. Therefore the pH is changed to more basic conditions, preferably to a pH of more than 8

The formation of triple helices is determined using segment B2 bound to molecule A, as a measure for the presence, absence or amount of molecule A. There are many ways to determine segment B2, depending upon the molecular structure of this segment. In a first instance segment B2 can be determined using its sequence of nucleobases. This sequence makes possible the use of further probes comprising a nucleobase sequence complementary to the sequence of segment B2.

In one embodiment, determination of segment B2 is by a probe containing a sequence complementary to segment B2 and a reporter group. Reporter groups are generally known as moieties that are itself detectable or can be detected by coupling to a detectable moiety. Examples of reporter groups are fluorescent moieties, like fluorescein, and enzymes, for example, peroxidase, or immunologically active substances, like haptens, for example, digoxigenin or colored substances, like rhodamine, or vitamins, like biotin. This labeled . probe will be termed secondary probe in the following. The secondary probes preferably do not contain a further nucleobase sequence complementary to a sequence contained in the reaction mixture and therefore are designed to stop the process of adding more probe molecules to molecule A.

The secondary probe can be added at any time to the reaction mixture or even to one of the reagents of the reaction. In a first embodiment, wherein the secondary probe is used as a blocker for the binding of further probe molecules, the secondary probe is added to the reaction mixture at the start of the incubation. The amount of secondary probe must be chosen such that it still allows enhancing the number of binding sites (for example B2) bound to molecule A, but also consecutive binding to a ratio of these binding sites. Therefore generally the amount of secondary probes will be less than the amount of probe molecules B. Secondary probes can further be used to control the level of amplification.

In a second embodiment the secondary probe is added to the reaction mixture after or before the incubation in order to stop the reaction. In this case the amount of the secondary probe is chosen such that it exceed the amount of free segments B2 likely be created during the incubation. This amount will depend upon the amount of molecules A contained in the mixture, the amount of probe molecules added to the mixture and the length of the incubation time.

The molecule A may then be determined via the label present in the secondary probe. This determination is performed analogous to the methods known in the art for these labels. The determination will preferably include calibration of the system by performing the identical reaction sequence for a reaction mixture containing known amounts of molecule A.

The present invention generally makes use of the idea that the formation of triple helix structures allows the formation of branched structures and thereby the formation of multiple sites for binding labels. The more probe molecules are assembled on one molecule A, the more binding sites are created. Using this principle there are various possibilities to assemble probes using the formation of triple helix structures, depending upon the nature of each of the two segments constituting the probe molecules. According to the invention it is possible to use only one kind of probe molecule, but it is also possible to combine different kinds of probe molecules. While the first approach will have the advantage that it is not necessary to synthesize probe molecules of different sequences, the second approach may have the advantage of the possibility to better adapt the assembling reaction to specific requirements. In the following there will be described one embodiment of each approach.

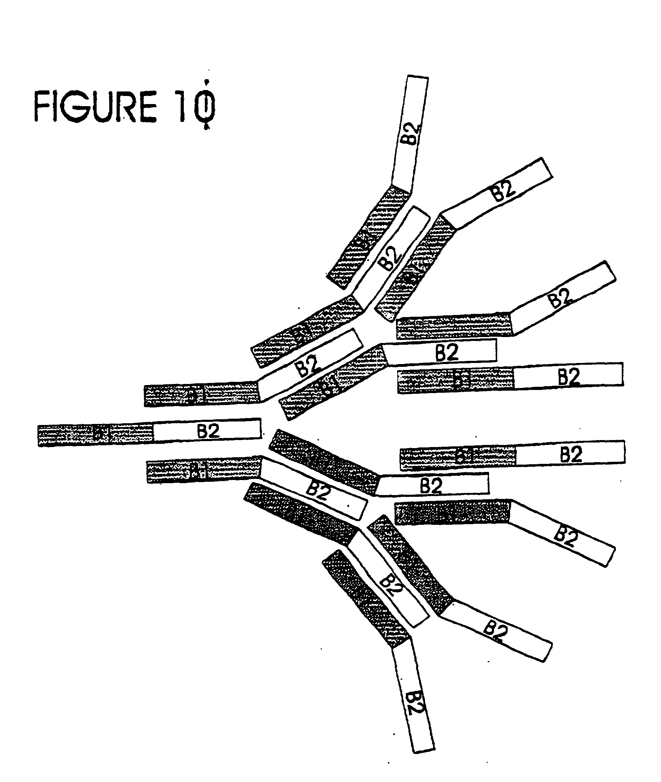

In a first embodiment only one kind of probe molecule is used. This probe molecule therefore constitutes probe molecule B. In Figure 10 a schematic structure of an assembly formed during incubation of multiple probe molecules B with a molecule A is shown. In this embodiment segment B1 is a component participating twice in the formation of a triplex and B2 is a component participating only once in this triple helix structure. An example for component B1 is PNA, while B2 can then be DNA. The sequence and orientation of the segments is chosen according to the principles laid down in Figures 2 and 3. It will be recognized that the longer the incubation time is, the larger the assembly grows and the more binding sites useful for the determination for example segments B2 are available, for example for binding a labeled secondary probe.

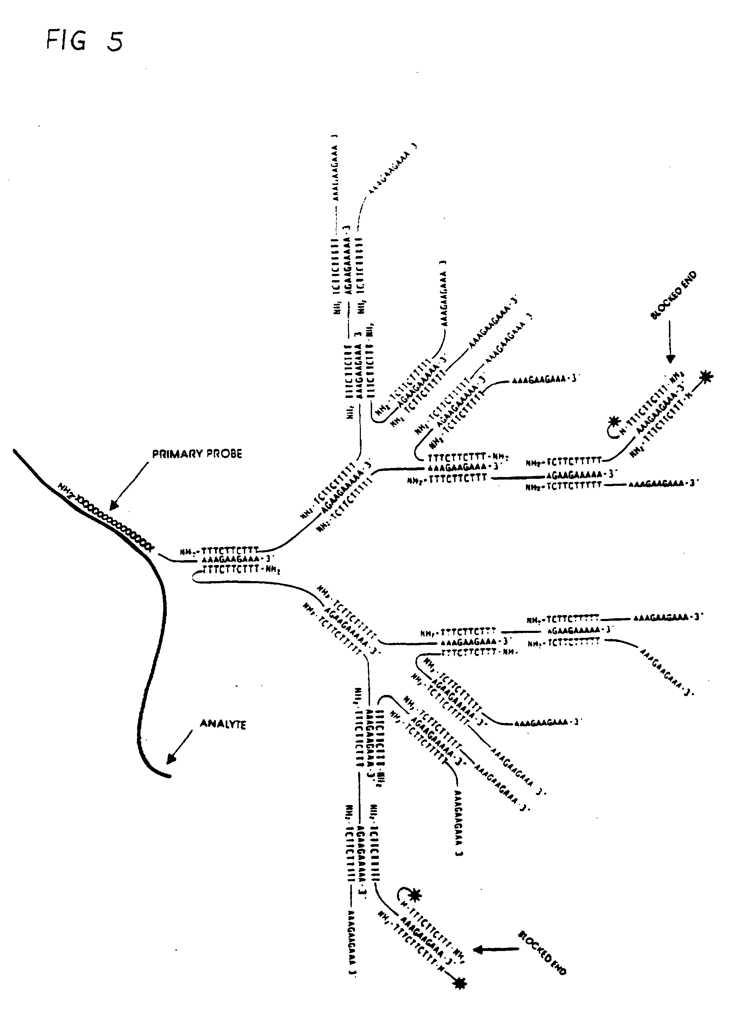

In a second embodiment which also uses chimera of one segment capable of occurring twice in triple helix structure and one segment occurring once in a triple helix structure, two kinds or probe molecules are used. These probe molecules differ in structure such that the sequences of segments 1 and 2 of each kind or probe are chosen such that these segments cannot form triple helices with the other segment of the same kind of probe. However, the second segment B2 of the first probe molecule B is capable of participating in triple helix structure with the first segment C1 of the second probe C and the second segment C2 of the second probe C can form a triple helix structure with the first segment B 1 of a further probe molecule B. During incubation of these probe molecules with molecule A an assembly will be formed that contains a branched structure wherein the probe molecules are incorporated in an alternating way. Such a structure is shown in Figure 5. Figure 5 in addition shows that secondary probes containing a segment complementary to one of segments B2 or C2 of the probe molecules B or C can be incorporated to put a label on the assembly when the secondary probe acts as a terminator of the assembly process. The secondary probe should then not contain another segment complementary to any other segment of probe molecules B and C. This is named by the term blocked end. Figure 5 further shows the formation of triplexes of different orientation, depending upon the sequence of the probe molecules.

As can be seen further, the segments preferably contain either purines or pyrimidines, such that one segment contains only purines, while the other contains only pyrimidines. This is preferred because highly stable triple helices form when pyrimidines of a PNA base pair to purines of a DNA using Watson/Crick binding and Hoogsteen binding.

The segments of the probe molecules can be synthesized independently, especially if the segments comprise different backbones. The synthesis of PNAs is described in WO 92/20702, whereas the synthesis of deoxyribonucleotides is possible according to a wide variety of methods, comprising chemical synthesis via phosporamidites or, especially for longer sequences, by methods including enzymes, like in the template dependent synthesis or restriction of double stranded nucleic acids. In a subsequent step the two segments are connected by the linker moiety L. The linker can be any molecular unit, such as amino acid residues, e.g., aspartate or glutamate, or other compounds like 8-amino-dioxa-octanoic acid (Ado, according to DE-A-3943522) or hexamethylene.

In a third embodiment, which can involve both, duplex formation and triplex formation, there is used a first kind of probes containing first and second segments which occur twice in a triplex structure, whereas a second kind of probe molecules contains first and second segments that occur only once in triplex structures. In one example probe B contains two PNA segments and probe C contains two DNA segments. The sequence of segment C1 is such that it binds segment B2 and the sequence of segment C2 is chosen such that it binds sequence B1 of an additional molecule of probe B. Again, as outlined above. the sequences are chosen in sequence and symmetry such that the probes cannot bind intramolecularly and such that the overall sequence of probe B is not complementary to overall sequence of probe C. An assembly of those probes B and C leads to alternating assembly of the probes. The synthesis of such probes can be especially advantageous because they can be synthesized without interrupting automated chemical synthesis.

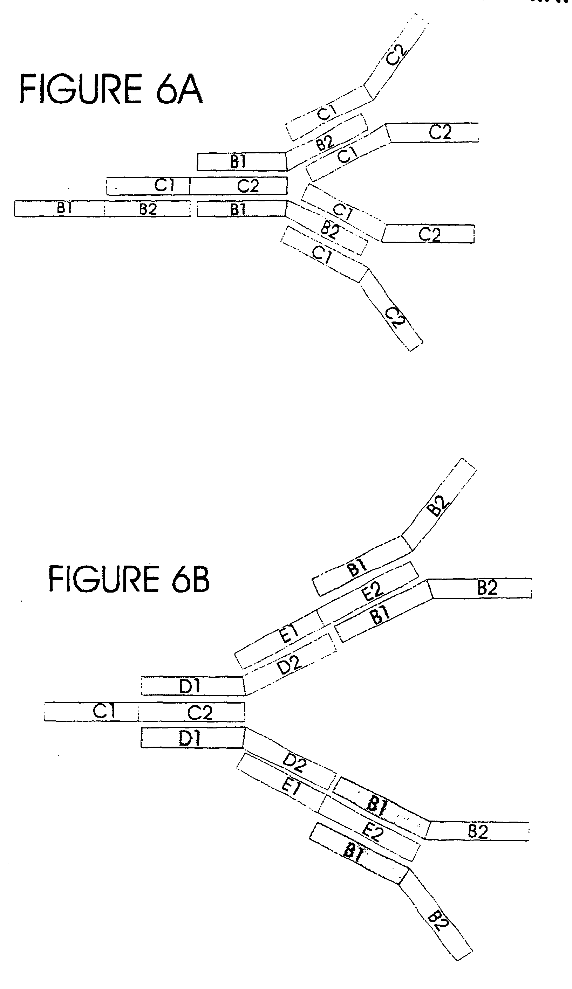

In case the assembly reaction involves four or more compounds (as in Figure 6B), some compounds (for example compounds B and C as well as D and E or B, C and D or C, D, and E) may be combined and reacted prior to the overall mixing. Thus the duplex- or triplex-hybridisations may be conducted separately. Alternatively, each compound may be added stepwise, each step being succeeded by a washing step.

As described above for the chimeric molecules, it is possible to construct assemblies with more probes. An example of such a construct is given in Figure 6B. In this case segment B2 of probe B is complementary to a segment C1 of probe C, segment C2 is complementary to a first segment D1 of a further kind of probe D, which in addition contains a segment D2 which is complementary to a first segment E1 of a further kind of probe molecule E, which in turn contains a segment E2 complementary to the sequence of segment B1. This assembly requires that for example segment C2 is not complementary to a segment B 1 and D1 and segment C2 is not complementary to segment B1 and C1. In this way a controlled assembly of a multiplicity of probes using triple helix structures is possible.

The use of probe molecules containing only one kind of backbone in both segments has the advantage of easier synthesis of the probe molecules. The probe molecules can easily be synthesized totally from monomeric units without the need of a subsequent linkage step. If it is desired to use a linker connecting segments 1 and 2, or if an opposing orientation of the segments is chosen, it may be advantageous to synthesize the segments independently and connect them in a subsequent step as pointed out above.

A further subject of the invention is a molecule B containing a nucleobase sequence containing segments B1 and B2 wherein B1 contains a peptide bond containing backbone and B2 contains a natural backbone and wherein said segments are covalently bound together directly or through a linker moiety and wherein said binding is made between the amino terminus of B1 and the 3' terminus of B2 or the carboxylic acid terminus of B1 and the 5' terminus of B2. A peptide bond according to the invention is the chemical bond between a group CO and a group NR, wherein R is hydrogen or C₂-C₆ acyl or C₁-C₆ alkyl. A natural backbone contains sugar-phosphate units.

A further subject of the invention is a method for the determination of an analyte comprising providing a reaction mixture containing a nucleobase SA containing molecule A, said molecule being capable of forming a triple helix structure through said sequence and being capable of binding to said analyte multiple probe molecules B containing a first segment B1 capable of participating in a triple helix structure with A through said sequence SA and a second segment B2 incubating said reaction mixture at conditions allowing the formation of triple helices and determining the formation of triple helices using said segment B2 as a measure for the analyte to be determined. Preferably the molecule A is a probe present in the reaction mixture in an amount dependent upon the presence/absence or amount of said analyte. An analyte according to this method is any molecule desired to be determined. Typical analytes are constituents of body fluids, for example blood or fluids derived therefrom, like serum or plasma, or urine or sputum. Some fluids require preparational steps to bring the analytes into accessible form. For example could it be required to lyse cell walls in order to release analytes. The analytes can be for example antibodies, antigens, haptens or nucleic acids. Nucleic acids are preferred analytes. In order to obtain a flexible system for universal amplification it is preferred to use a primary probe comprising a first segment of nucleobases complementary to the sequence of a stretch of the analyte sequence and a second segment which contains a nucleobase sequence complementary to segment B1. In this system the probe molecules can be used for analytes having different nucleobase sequences. A construct formed is shown in Figure 6.

A further subject of the invention is the use of formation of multiple triple helix structures for the determination of an analyte.

A further subject of the invention is a composition of matter comprising a component N containing a sequence N1 capable of forming a triplex structure and a sequence N2 accessible for further hybridization and a component M containing a sequence M1 capable of forming a triplex structure including sequence N1 and a sequence M2 accessible for further hybridization. Examples of components N and M are molecule A and probe B or probe B and probe C. Those compositions of matter are intermediate products in the assembly of the above process. However, they can be used further to determine nucleic acids. An example of component O is probe D.

The following examples are given to exemplify the invention and determine conditions for successful conduction of the method:

### General

The PNAs were synthesized according to WO 92/20702. If applicable, modifying groups are attached while the PNAs were still protected and on the solid phase. The PNAs had an amide function at the COOH end because of the choise of solid support and the subsequent way to decouple the PNA from the solid phase. Thus, in this case we denote the C-end with - CONH₂. The amino end is denoted -H. Monomers for synthesis of the PNAs were purchased from Millipore, USA. Oligonucleotides were synthesesized chemically on an automated synthesizer.

### Example 1

### Analysis of conditions for triplex formation

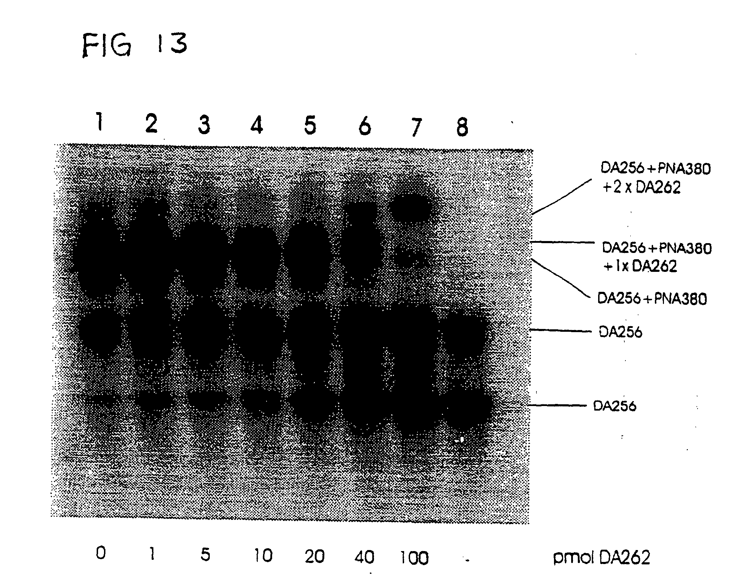

In order to estimate the of triplex forming capability of oligos (for example PNAs or probes B and C) melting temperature Tm was measured at pH 4.5 and 9.5, since no Hoogsteen base pairing is possible at the higher pH. In total, 1 ml of a solution containing 0.1 M phosphate buffer at the desired pH .Nine µM PNA and 4.5 µM DNA was heated to 95°C for 10 min., and allowed to cool slowly at 22°C for 18 h. Subsequently, 2 ml 0.1 M phosphate buffer at the relevant pH was added, and the optical density was measured ramping the temperature from 25°C to 95°C at 0.5°C/min. As can be seen from Figure 12, Tm of segments B1 and C2 is 90°C at pH 4.5, and 58 °C at pH 9.5. Such pH-dependency shows that Hoogsteen base pairing contributes significantly to the stability of the hybridisation complex, and that triplex formation has occurred.

The sequences of segments B1 (PNA) and C2 (DNA) are

### Example 2

### Synthesis of DNA-PNA chimera

A DNA oligo with a six-carbon atom (hexamethylene) linker and a terminal, was linked to a PNA oligo with glutamate residue at the amino terminus, i.e., the DNA oligo SIG1 with PNA187 and the DNA oligo SIG2 with PNA 184. The reaction mixture was as follows:
5 µl DMSO
2 µl EDC, 0.5 M
2 µl imidazole, 1 M
3.6 nmol PNA (PNA187 or PNA184)
3.6 nmol DNA (SIG1 or SIG2)
H₂O to 20 µl
where:

The reaction proceeded at 22°C for 1-5 days.

Chimera with the following sequences were produced:

The DNA was labelled with ³²P-ddATP by convential enzymatic terminal elongation.

### Example 3

### Analysis of chimera by gel chromatography

After reaction, 30 µl was added 5 µl 80% formamide, heated to 95°C for 5 min., cooled on wet ice for 10 min., and applied a 12% denaturing polyacrylamide gel.
18 ml 20% gelmix
10.8 ml 8% urea
1.2 ml 10 x TBE (0.9 M Tris-borate, 0.01 M EDTA)
300 µl APS (ammonium per sulphate)
10 µl TEMED (N,N,N',N'-tetramethylethylene diamine)
where 20% gelmix consists of 7 M urea, 90 mM tris-borate pH 8.3 and 0.67% bisacrylamid. Electrophoresis was performed for 60 min. at 400 V. After electrophoresis the gel was wrapped in polyethylene film, placed on TLC plate, subjected to UV light and photographed. A reaction product was observed with approximately half the migration rate the DNA oligos.

### Example 4

### Purification of chimera

After electrophoresis, a gel fragment containing the chimera was excised from the gel, washed in 1 ml TE Buffer for 5 min., subsequently placed into 200 µl TE pH 8.3, and extracted with vigorous shaking overnight at 55°C.

### Example 5

### Formation of assemblies for signal amplification

In order to show that complexes of DNA with PNA can be formed wherein both, a part of the DNA and a part of the PNA is accessible for binding further probe molecules, the following PNA and DNA molecules were incubated:

The results are shown in Fig 12.

### Experiment 1:

This is just the preparation of a solution containing 1 pmol DA256* (see lane 1).

### Experiment 2:

In this experiment 1 pmol DA256* and 10 pmol PNA414 were mixed in 10 µl water and incubated overnight (see lane 2).

### Experiment 3:

In this experiment 1 pmol DA256* and 10 pmol PNA380 were incubated. This experiment yields a DNA having two PNAs bound via triplex formation. This is the first molecule that can be used as a probe in nucleic acid hybridisation assays, if the part of the DNA not participating in the triplex formation is complementary to a selected region of a target nucleic acid. Instead of one binding side, now two binding sites (the free accessible parts of the PNAs) have been created (see lane 3).

### Experiment 4:

In this experiment two DNA molecules (1 pmol DA256* and 20 pmol DA262) are incubated together with 10 pmol PNA380. This experiment shows that the binding sides remaining on the PNA380 further react with DNA molecules of complementary sequence forming duplexes. This can be seen from lane 4 of Fig 12, wherein the complex formed as seen in lane 3 disappears in favour of higher molecular weight bands.

### Experiment 5:

This experiment shows that also the DNA part of DA256 is accessible to hybridisation with a PNA molecule, forming a duplex. In this experiment 1 pmol DA256*, 5 pmol PNA414 and 10 pmol PNA380 are mixed and incubated overnight. In lane 5 of Fig 5 it can be seen that, compared to lane 3, there is formed a higher molecular weight complex, indicating additional duplex formation with PNA414.

### Experiment 6:

In this experiment all four probes were mixed together to create very high molecular weight assemblies. 1 pmol DA256*, 9 pmol DA256, 10 pmol DA262, 10 pmol PNA380 and 10 pmol PNA414 are mixed and incubated overnight. As can be seen from lane 6 of Fig 13 high molecular weight structures have formed. This is the first indication that the binding sites of originally 1 probe is enhanced four times. The conditions for running the gel of Fig 12 is 12 % polyacrylamid with the buffer of TAE pH 7.0.

### Example 6

### Formation of an assembly having doubled binding sites

This experiment shows the formation of the complex containing a first probe (DA256) binding via triplex formation to further probes (PNA380), which bind via duplex formation one further probe (DA262) each. The sequences of the oligomers used are given in Example no. 6. The conditions are the following:

Samples 1-7 contain:
1 pmol DA 256*
9 pmol DA256
40 pmol PNA380
X pmol PNA 262 (X = 0; 1; 5; 10; 20; 40; 100)

Gel: 12 % Polyacrylamid
Buffer: TAE pH 7.0

The results are presented in Fig 13. The lanes correspond to the samples as given above. It can be seen with lower concentrations of DA262 there is formed predominant an assembly of DA256 and PNA380 having a higher molecular weight than DA256. With increasing amount of DA262 there is formed a higher molecular weight complex (especially lanes 6 and 7). In lane 7 the predominant product is the complex containing one triplex structure and two duplex structures.

## Claims

1. A method of determining a molecule A containing a sequence SA of nucleobases, said molecule being capable of participating in a triple helical structure through said sequence SA comprising
- providing a reaction mixture containing multiple probe molecules B containing a first segment B1 capable of participating in a triple helix structure with A through sequence SA and a segment B2,
- incubating said reaction mixture at conditions resulting in the formation of branched triple helix structures and
- determining the formation of branched triple helix structures using said segment B2.

2. A method according to claim 1, **characterised in that** segment B2 is capable of participating in a triple helix structure.

3. A method according to claim 1, **characterised in that** segments B1 or/and B2 occur twice in said triple helix structure.

4. A method according to any of claims 1 to 3, **characterised in that** the determination of branched triple helix structures is made by means of a probe C containing
a first segment C1 which can bind to segment B2 and a second segment C2
wherein either or both of C1 and C2 are capable of participating in a triple helix structure
and determining the formation of triple helices using segment C2.

5. A method according to claim 4, **characterised in that** segment C2 is capable of participating in a triple helix structure.

6. A method according to claim 4, **characterised in that** segments C1 and/or C2 occur twice in a triple helix structure.

7. A method according to any of the preceding claims, **characterised in that** one of segments B1 and B2 contains a nucleobase sequence of a non-natural backbone and the other contains a nucleobase sequence of a natural backbone.

8. A method according to any of claims 4-6, **characterised in that** B1 and B2 contain a nucleobase sequence on a non-natural backbone and C1 and C2 contain a nucleobase sequence on a natural backbone.

9. A method according to any of claims 1 to 3, **characterised in that** the formation of branched triple helix structures are made using a labelled probe C containing a nucleobase sequence complementary to B2.

10. A method according to any of claims 7 and 8, **characterised in that** the non-natural backbone is a peptide bond containing backbone.

11. A method according to any of claims 1 to 10, wherein one or more additional multiple probe molecules, D, E, are involved in the formation of multiple triple helix structures in form of branched triple helix structures, said additional probe molecules each containing a first segment which can bind to a segment of a different probe molecule and a second segment wherein either or both of said segments are capable of participating in a triple helix structure.

12. A molecule containing a nucleobase sequence containing segments B1 and B2, wherein B1 contains a peptide bond containing backbone and B2 contains a natural backbone, and wherein said segments are covalently bound together directly or through a linker moiety, and wherein said binding is between the amino terminus of B1 and the 5' terminus of B2 or the carboxylic acid terminus of B1 and the 3' terminus of B2, and wherein said segments are both capable of participating in triple helix structures.

13. A method for the determination of an analyte comprising
- providing a reaction mixture with a molecule A containing a nucleobase sequence SA, said molecule being capable of forming a triple helix structure through said sequence SA and further being capable of binding to said analyte and multiple probe molecules B containing
a first segment B1 capable of participating in a triple helix structure with A through said sequence SA and
a second segment B2
- incubating said reaction mixture at conditions resulting in the formation of branched triple helix structures and
- determining the formation of branched triple helix structures using said segment B2 as a measure for the analyte to be determined.

14. A method according to claim 13, **characterised in that** the incubation is stopped at a -predetermined time by changing the pH of the reaction mixture or by adding blocking molecules.

15. Use of a molecule A and a molecule B for the formation of branched triple helix structures for the determination of an analyte, wherein molecule A contains a nucleobase sequence SA being capable of forming a triple helix structure and further being capable of binding to said analyte and to said molecules B, molecule B containing a first segment B1 being capable of participating in a triple helix structure with A through said sequence SA and a second segment B2 capable of participating in a triple helix structure.

16. Use according to claim 15 wherein molecule B contains a nucleobasa sequence containing segments B1 and B2, wherein B1 contains a peptide bond containing backbone and B2 contains a natural backbone, and wherein said segments are bound together directly or through a linker moiety, and wherein said binding is between the amino terminus of B1 and the 5' terminus of B2 or the carboxylic acid terminus of B1 and the 3' terminus of B2.

17. Use of a molecule B and a molecule C for the formation of branched triple helix structures for the determination of a molecule A containing a sequence SA of nucleobases being capable of participating in a triple helical structure through said sequence SA, molecule B containing a first segment B1 capable of participating in a triple helix structure with A through sequence SA and a segment B2 capable of further hybriziding, optionally capable of participating in a triple helix structure, and said molecule C containing a first segment C1 which can bind to segment B2 and a second segment C2 which can bind segment B1 of a further probe B, said probe molecules capable of being incorporated in an alternating way.

## Patentansprüche

1. Verfahren zum Bestimmen eines Moleküls A mit einer Sequenz SA von Nukleobasen, wobei sich das Molekül durch die Sequenz SA an einer Dreifachhelixstruktur beteiligen kann, umfassend
- Bereitstellen einer Reaktionsmischung mit mehreren Sondenmolekülen B mit einem ersten Segment B1, das sich an einer Dreifachhelixstruktur mit A durch Sequenz SA beteiligen kann, und einem Segment B2,
- Inkubieren der Reaktionsmischung bei Bedingungen, die zur Bildung von verzweigten Dreifachhelixstrukturen führen, und
- Bestimmen der Bildung von verzweigten Dreifachhelixstrukturen mithilfe des Segments B2.

2. Verfahren nach Anspruch 1, **dadurch gekennzeichnet, dass** Segment B2 sich an einer Dreifachhelixstruktur beteiligen kann.

3. Verfahren nach Anspruch 1, **dadurch gekennzeichnet, dass** Segment B1 und/oder B2 in der Dreifachhelixstruktur zweimal vorkommen.

4. Verfahren nach einem der Ansprüche 1 bis 3, **dadurch gekennzeichnet, dass** die Bestimmung von verzweigten Dreifachhelixstrukturen mithilfe einer Sonde C erfolgt, umfassend
ein erstes Segment C1, das an Segment B2 binden kann, und ein zweites Segment C2,
wobei sich entweder C1 oder C2 oder beide an einer Dreifachhelixstruktur beteiligen können,
und Bestimmen der Bildung von Dreifachhelices mithilfe von Segment C2.

5. Verfahren nach Anspruch 4, **dadurch gekennzeichnet, dass** sich Segment C2 an einer Dreifachhelixstruktur beteiligen kann.

6. Verfahren nach Anspruch 4, **dadurch gekennzeichnet, dass** Segment C1 und/oder C2 in einer Dreifachhelixstruktur zweimal vorkommen.

7. Verfahren nach einem der vorhergehenden Ansprüche, **dadurch gekennzeichnet, dass** eines der Segmente B1 und B2 eine Nukleobasensequenz auf einem nicht natürlichen Gerüst enthält und das andere eine Nukleobasensequenz auf einem natürlichen Gerüst enthält.

8. Verfahren nach einem der Ansprüche 4 - 6, **dadurch gekennzeichnet, dass** B1 und B2 eine Nukleobasensequenz auf einem nicht natürlichen Gerüst enthalten und C1 und C2 eine Nukleobasensequenz auf einem natürlichen Gerüst enthalten.

9. Verfahren nach einem der Ansprüche 1 bis 3, **dadurch gekennzeichnet, dass** die Bildung von verzweigten Dreifachhelixstrukturen mithilfe einer markierten Sonde C erfolgt, die eine zu B2 komplementäre Nukleobasensequenz enthält.

10. Verfahren nach einem der Ansprüche 7 und 8, **dadurch gekennzeichnet, dass** das nicht natürliche Gerüst ein Gerüst mit Peptidbindungen ist.

11. Verfahren nach einem der Ansprüche 1 bis 10, wobei ein zusätzliches multiples Sondenmolekül bzw. mehrere zusätzliche multiple Sondenmoleküle, D, E, an der Bildung von mehreren Dreifachhelixstrukturen in Form von verzweigten Dreifachhelixstrukturen beteiligt sind, wobei die zusätzlichen Sondenmoleküle jeweils ein erstes Segment enthalten, das an ein Segment eines anderen Sondenmoleküls binden kann, und ein zweites Segment, wobei sich eines der Segmente bzw. beide Segmente an einer Dreifachhelixstruktur beteiligen kann bzw. können.

12. Molekül mit einer Nukleobasensequenz mit Segmenten B1 und B2, wobei B1 ein Gerüst mit Peptidbindungen enthält und B2 ein natürliches Gerüst enthält und wobei die Segmente entweder direkt oder über eine Linkerhälfte kovalent aneinander gebunden sind und wobei die Bindung zwischen dem Aminoterminus von B1 und dem 5'-Terminus von B2 vorliegt bzw. dem Carboxylsäureterminus von B1 und dem 3'-Terminus von B2 vorliegt und wobei sich die Segmente beide an Dreifachhelixstrukturen beteiligen können.

13. Verfahren zum Bestimmen eines Analyten, umfassend
- Bereitstellen einer Reaktionsmischung mit einem Molekül A mit einer Nukleobasensequenz SA, wobei das Molekül durch die Sequenz SA eine Dreifachhelixstruktur bilden kann und an den Analyten und mehrere Sondenmoleküle B, umfassend
ein erstes Segment B1, das sich an einer Dreifachhelixstruktur mit A durch die Sequenz SA beteiligen kann, und
ein zweites Segment B2, binden kann,
- Inkubieren der Reaktionsmischung bei Bedingungen, die zur Bildung von verzweigten Dreifachhelixstrukturen führen, und
- Bestimmen der Bildung von verzweigten Dreifachhelixstrukturen mithilfe des Segments B2 als ein Maß für den zu bestimmenden Analyten.

14. Verfahren nach Anspruch 13, **dadurch gekennzeichnet, dass** die Inkubation zu einem vorbestimmten Zeitpunkt durch Verändern des pH-Wertes der Reaktionsmischung oder durch Zugabe von Blockierungsmolekülen angehalten wird.

15. Verwendung eines Moleküls A und eines Moleküls B für die Bildung von verzweigten Dreifachhelixstrukturen für die Bestimmung eines Analyten, wobei Molekül A eine Nukleobasensequenz SA enthält, die sich an der Bildung einer Dreifachhelixstruktur beteiligen und des Weiteren an den Analyten und an die Moleküle B binden kann, wobei Molekül B ein erstes Segment B1 enthält, das sich an einer Dreifachhelixstruktur mit A durch die Sequenz SA beteiligen kann, und ein zweites Segment B2, das sich an einer Dreifachhelixstruktur beteiligen kann.

16. Verwendung nach Anspruch 15, wobei Molekül B eine Nukleobasensequenz mit Segmenten B1 und B2 enthält, wobei B1 ein Gerüst mit Peptidbindungen enthält und B2 ein natürliches Gerüst enthält, und wobei die Segmente entweder direkt oder indirekt über eine Linkerhälfte aneinander gebunden sind und wobei die Bindung zwischen dem Aminoterminus von B1 und dem 5'-Terminus von B2 bzw. dem Carboxylsäureterminus von B1 und dem 3'-Terminus von B2 vorliegt.

17. Verwendung eines Moleküls B und eines Moleküls C für die Bildung von verzweigten Dreifachhelixstrukturen für die Bestimmung eines Moleküls A mit einer Sequenz SA von Nukleobasen, das sich durch die Sequenz SA an Dreifachhelixstrukturen beteiligen kann, wobei Molekül B ein erstes Segment B1 enthält, das sich an einer Dreifachhelixstruktur mit A durch Sequenz SA beteiligen kann, und ein Segment B2, das zur weiteren Hybridisierung in der Lage ist, sich optional an einer Dreifachhelixstruktur beteiligen kann, und wobei das Molekül C ein erstes Segment C1 enthält, das an Segment B2 binden kann, und ein zweites Segment C2, das Segment B1 einer weiteren Sonde B binden kann, wobei die Sondenmoleküle auf alternative Weise eingebaut sein können.

## Revendications

1. Méthode pour déterminer une molécule A contenant une séquence SA de bases nucléiques, ladite molécule étant capable de participer à une structure en triple hélice par l'intermédiaire de la séquence SA, comprenant
- la fourniture d'un mélange réactionnel contenant plusieurs molécules sondes B contenant un premier segment B1 capable de participer à une structure en triple hélice avec A par l'intermédiaire de la séquence SA et un segment B2,
- l'incubation dudit mélange réactionnel dans des conditions conduisant à la formation de structures en triple hélice ramifiées et
- la détermination de la formation de structures en triple hélice ramifiées à l'aide dudit fragment B2.

2. Méthode selon la revendication 1, **caractérisée en ce que** le segment B2 est capable de participer à une structure en triple hélice.

3. Méthode selon la revendication 1, **caractérisée en ce que** les segments B1 et/ou B2 sont présents deux fois dans ladite structure en triple hélice.

4. Méthode selon l'une des revendications 1 à 3, **caractérisée en ce que** la détermination de structures en triple hélice ramifiées est réalisée à l'aide d'une sonde C contenant
un premier segment C1 qui peut se lier au segment B2 et
un deuxième segment C2
dans laquelle un seul ou les deux d'entre C1 et C2 sont capables de participer à une structure en triple hélice
et la formation de triples hélices est déterminée à l'aide du segment C2.

5. Méthode selon la revendication 4, **caractérisée en ce que** le segment C2 est capable de participer à une structure en triple hélice.

6. Méthode selon la revendication 4, **caractérisée en ce que** les segments C1 et/ou C2 sont présents deux fois dans une structure en triple hélice.

7. Méthode selon l'une des revendications précédentes, **caractérisée en ce qu'**un des segments B1 et B2 contient une séquence de bases nucléiques sur un squelette non naturel et l'autre contient une séquence de bases nucléiques sur un squelette naturel.

8. Méthode selon l'une des revendications 4 à 6, **caractérisée en ce que** B1 et B2 contiennent une séquence de bases nucléiques sur un squelette non-naturel et C1 et C2 contiennent une séquence de bases nucléiques sur un squelette naturel.

9. Méthode selon l'une des revendications 1 à 3, **caractérisée en ce que** la formation de structures en triple hélice ramifiées est réalisée à l'aide d'une sonde marquée C contenant une séquence en bases nucléiques complémentaire à B2.

10. Méthode selon l'une des revendications 7 et 8, **caractérisée en ce que** le squelette non naturel est un squelette coiltenant des liaisons peptidiques

11. Méthode selon l'une des revendications 1 à 10, dans laquelle une ou plusieurs molécules sondes multiples additionnelles, D, E, sont impliquées dans la formation de structures en triple hélice multiples sous la forme de structures en triple hélice ramifiées, lesdites molécules sondes additionnelles contenant chacune un premier segment qui peut se lier à un segment d'une molécule sonde différente et un deuxième segment, dans lesquelles un ou les deux desdits segments sont capables de participer à une structure en triple hélice.

12. Molécule contenant une séquence de bases nucléiques contenant les segments B1 et B2, dans laquelle B1 contient un squelette contenant des liaisons peptidiques et B2 contient un squelette naturel, et dans laquelle lesdits segments sont liés ensemble de manière covalente directement ou par un fragment de liaison, et dans laquelle ladite liaison se trouve entre l'extrémité aminé de B1 et l'extrémité 5'de B2 ou l'extrémité acide carboxylique de B1 et l'extrémité 3' de B2, et dans laquelle lesdits segments sont tous deux capables de participer à une structure en triple hélice.

13. Méthode pour la détermination d'un composé à analyser comprenant
- la fourniture d'un mélange réactionnel comprenant une molécule A contenant une séquence de bases nucléiques SA, ladite molécule étant capable de former une structure en triple hélice par l'intermédiaire de ladite séquence SA et étant de plus capable de se lier audit composé à analyser, et des molécules sondes multiples B contenant
un premier segment B1 capable de participer à une structure en triple hélice avec A par l'intermédiaire de ladite séquence SA et
un deuxième segment B2
- l'incubation dudit mélange réactionnel dans des conditions conduisant à la formation de structures en triple hélice ramifiées et
- la détermination de la formation de structures en triple hélice à l'aide dudit segment B2 comme mesure du composé à analyser devant être déterminé.

14. Méthode selon la revendication 13, **caractérisée en ce que** l'incubation est arrêtée à un temps prédéterminé en changeant le pH du mélange réactionnel ou en ajoutant des molécules bloquantes.

15. Utilisation d'une molécule A et d'une molécule B pour la formation de structures en triple hélice ramifiées pour la détermination d'un composé à analyser, dans laquelle la molécule A contient une séquence de bases nucléiques SA capable de former une structure en triple hélice et de plus capable de se lier audit composé à analyser et auxdites molécules B, la molécule B contenant un premier segment B1 capable de participer à une structure en triple hélice avec A par l'intermédiaire de ladite séquence SA et un deuxième segment B2 capable de participer à une structure en triple hélice.

16. Utilisation selon la revendication 15, dans laquelle la molécule B contient une séquence de bases nucléiques contenant les segments B1 et B2, dans laquelle B1 contient un squelette contenant des liaisons peptidiques et B2 contient un squelette naturel, et dans laquelle lesdits segments sont liés ensemble directement ou par l'intermédiaire d'un fragment de liaison, et dans lequel ladite liaison se trouve entre l'extrémité aminé de B1 et l'extrémité 5' de B2 ou l'extrémité acide carboxylique de B1 et l'extrémité 3' de B2.

17. Utilisation d'une molécule B et d'une molécule C pour la formation de structures en triple hélice ramifiées pour la détermination d'une molécule A contenant une séquence SA de bases nucléiques capable de participer à une structure en triple hélice par l'intermédiaire de la dite séquence SA, la molécule B contenant un premier segment B1 capable de participer à une structure en triple hélice avec A par l'intermédiaire de la séquence SA et un segment B2 capable d'hybridations supplémentaires, éventuellement capable de participer à une structure en triple hélice, et ladite molécule C contenant un premier segment C1 qui peut se lier au segment B2 et un deuxième segment C2 qui peut se lier au segment B1 d'une sonde B supplémentaire, lesdites molécules sondes étant capables d'être incorporées d'une manière différente.


### SEQUENCE LISTING

(1) GENERAL INFORMATION:
   (i) APPLICANT:
      (A) NAME: Boehringer Mannheim GmbH
      (B) STREET: Sandhofer Straße 116
      (C) CITY: Germany
      (E) COUNTRY: DE
      (F) POSTAL CODE (ZIP): 68305
   (ii) TITLE OF INVENTION: Method of analysis using signal amplification
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Oligodeoxyribonucleotide"
   (ix) FEATURE:
      (A) NAME/KEY: misc_feature
      (B) LOCATION: 1
      (D) OTHER INFORMATION:/note= "at the terminus aminohexyl alkylated amino group"
   (xi) SEQUENCE DESCRIPTION: SEQ ID NO: 1:
   AAAGAAGAAA 10
(2) INFORMATION FOR SEQ ID NO: 2:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Oligodeoxyribonucleotide"
   (ix) FEATURE:
      (A) NAME/KEY: misc_feature
      (B) LOCATION:1
      (D) OTHER INFORMATION:/note= "at the terminus aminohexyl alkylated amino group"
   (xi) SEQUENCE DESCRIPTION: SEQ ID NO: 2:
   AAGAGAAAAA 10
(2) INFORMATION FOR SEQ ID NO: 3:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Oligodeoxyribonucleotide"
   (xi) SEQUENCE DESCRIPTION: SEQ ID NO: 3:
   CGACTTCAGC ATGACTCAAA GAAGAAA 27
(2) INFORMATION FOR SEQ ID NO: 4:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Oligodeoxyribonucleotide"
   (xi) SEQUENCE DESCRIPTION: SEQ ID NO: 4:
   ATGTGGACAT TGTCATCAAG AAAAGAA 27